# FASCICULE DE BREVET EUROPEEN

(11) **EP 0 993 283 B1**
(45) Date de publication et mention de la délivrance du brevet: **08.12.2004**
(21) Numéro de dépôt: 98933694.6
(22) Date de dépôt: 23.06.1998
(51) Int. Cl.: A61F 5/01

(54) **DISPOSITIF POUR ETABLIR DES PRESSIONS APPLIQUEES PAR UNE ORTHESE COMPRESSIVE**
VORRICHTUNG ZUM FESTSTELLEN DES ANGEWANDTEN DRUCKES EINER KOMPRESSIONSORTHESE
DEVICE FOR MEASURING PRESSURE POINTS TO BE APPLIED BY A COMPRESSIVE ORTHOTIC DEVICE

(30) Priorité: 23.06.1997 FR 9707787
(43) Date de publication de la demande: 19.04.2000
(73) Titulaire: Innothera Topic International, 94110 Arcueil (FR)
(72) Inventeur: TESTUD, Jean-Louis, F-75013 Paris (FR); SENNOUNE, Mohammed, F-27000 Evreux (FR); PRUDHOMME, Jean-Pierre, F-92160 Antony (FR); OUCHENE, Amina, F-94700 Maisons Alfort (FR)
(74) Mandataire: Dupuis-Latour, Dominique
(86) Numéro de dépôt international: PCT/FR1998/001322
(87) Numéro de publication internationale: WO 1998/058605

(56) Documents cités:
- EP-A- 0 088 860
- US-A- 3 818 756
- US-A- 4 137 763
- US-A- 4 417 401
- US-A- 4 584 625

## Description

L'invention concerne la mesure des pressions de contention qu'une orthèse compressive permet d'appliquer sur une partie du corps.

L'invention sera plus particulièrement décrite dans le cas où la partie du corps considérée est la jambe et où l'orthèse compressive est un bas de contention. L'invention n'est cependant pas limitée à ce cas particulier, et s'applique à d'autres types d'orthèses et/ou d'autres parties du corps, par exemple des bandes élastiques de contention à appliquer sur la jambe ou une partie de la jambe, une ceinture de contention pour l'application d'une pression sur l'abdomen, etc.

Les pressions exercées par ces orthèses sont faibles, de l'ordre de 0 à 100 hPa, typiquement de l'ordre de 20 à 70 bPa, en pression relative.

De nombreux facteurs peuvent influer sur la valeur de cette pression et introduire des écarts par rapport à une valeur nominale standard, par exemple les réglages des machines de tricotage, les tolérances de fabrication, un traitement tel que la teinture du bas, etc.

Il est donc nécessaire de pouvoir mesurer de façon précise et reproductible la pression que permet réellement d'appliquer une orthèse compressive donnée, notamment pour vérifier sa conformité aux valeurs nominales (contrôle de qualité en fabrication).

Jusqu'à présent, cette mesure est effectuée en plaçant le bas à contrôler sur un gabarit en bois dont la forme et les dimensions sont normalisées ("modèle Hohenstein" de taille 1, 2, 3 ou 4), en glissant entre le bas et le gabarit une capsule mince en caoutchouc formant capteur manométrique (dispositif dénommé "Compritest") et en relevant la pression donnée par la capsule, d'abord sans le bas, puis avec celui-ci. La valeur recherchée est obtenue par différence entre ces deux valeurs.

Le US-A-4 137 763 décrit un dispositif de mesure utilisant de tels capteurs manométriques.

Cette méthode présente trois inconvénients majeurs :
- en premier lieu, sa précision est médiocre compte tenu, d'une part, de la pression relativement faible appliquée par le bas par rapport à la sensibilité de la capsule manométrique et, d'autre part, du fait que la mise en place du capteur entre le bas et le gabarit modifie la tension du bas à l'endroit de la mesure et fausse donc celle-ci ;
- la mesure est délicate et très dépendante de l'habilité de l'opérateur, car il est nécessaire de glisser la capsule entre le bas et le gabarit en déplaçant le moins possible le bas : la reproductibilité du procédé est donc difficile à assurer ;
- enfin, ce procédé ne donne qu'une seule mesure locale et pour avoir un autre point de mesure il est nécessaire de recommencer l'opération (mise en place de la capsule) autant de fois que l'on souhaite de points de mesure.

L'invention a pour but de remédier à ces inconvénients, en proposant un dispositif permettant d'établir une véritable cartographie des pressions susceptibles d'être appliquées par une orthèse compressive sur une partie du corps, et qui présente les avantages suivants :
- précision de la mesure ;
- fidélité de la mesure, afin de fournir des données de manière reproductible et indépendante de l'habilité d'un opérateur ;
- mesures simultanées (ou quasi-simultanées, en cas de multiplexage par exemple) en un grand nombre de points, de manière à obtenir un maillage anatomiquement représentatif de la carte des pressions appliquées par l'orthèse compressive ;
- simplicité et rapidité de mise en oeuvre ;
- possibilité de numériser, mémoriser, traiter et afficher les différentes données relevées, notamment pour permettre un interfaçage avec un traitement informatique.

A cet effet, le dispositif de l'invention comprend les caractéristiques définies dans la revendication 1.

Selon un certain nombre de caractéristique avantageuses :
- chacun des capteurs comprend à l'endroit du point de mesure, un diaphragme susceptible d'une microdéformation sous l'effet de la pression appliquée par l'orthèse, et des moyens de mesure, notamment à pont de jauges extensométriques compensées en température, de cette microdéformation ;
- le diaphragme fait partie d'une pastille support rapportée, incorporée à la forme de manière que sa surface extérieure, qui comprend le diaphragme, affleure celle de la forme ;
- le dispositif comprend en outre des moyens d'étalonnage de la pluralité de capteurs, avec une enceinte close étanche pressurisable enfermant la forme ;
- le dispositif comprend en outre des moyens de traitement et d'affichage des mesures recueillies par la pluralité de capteurs.

On va maintenant décrire un exemple de réalisation de l'invention, en référence aux figures annexées.
La figure 1 est une vue schématique, en élévation et partiellement en coupe, du dispositif de l'invention relié à un ordinateur pour l'exploitation des données mesurées.
La figure 2 est une vue agrandie du détail repéré II sur la figure 1 montrant la structure de l'un des capteurs du dispositif.
La figure 3 est une vue en coupe selon III-III de la figure 2.
La figure 4 est un schéma par blocs montrant les circuits électroniques de traitement des signaux de mesure fournis par les capteurs du dispositif de l'invention.

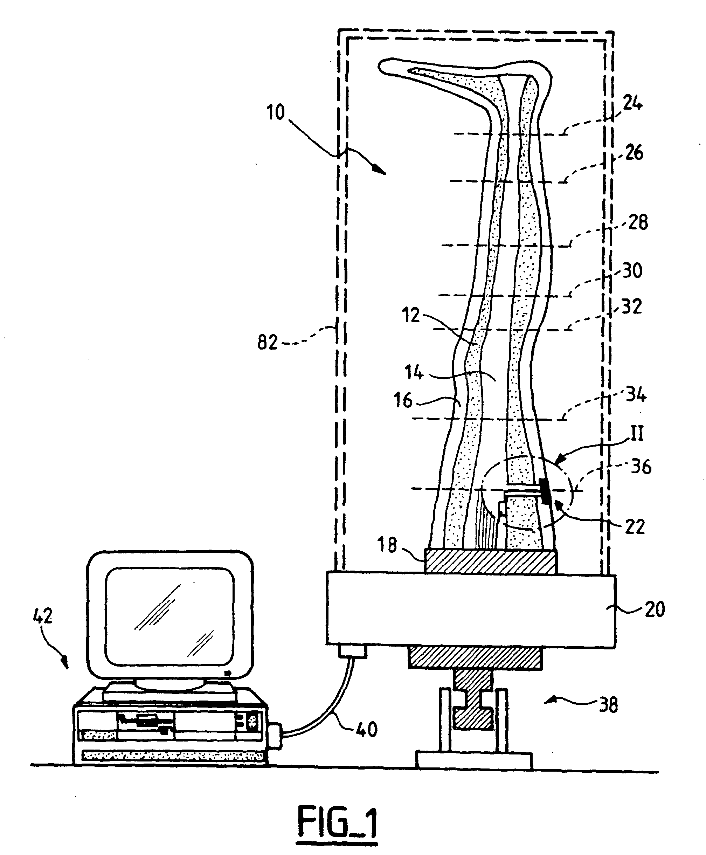

Sur la figure 1, la référence 10 désigne, de façon générale, une forme rigide représentative du membre (ou, de façon générale, de la partie du corps) sur lequel doit être appliquée la contention. Dans l'exemple illustré, il s'agit d'une forme de jambe dont les dimensions sont conformes au "modèle Hohenstein" normalisé de l'une des quatre tailles 1 à 4.

Cette forme 10 comporte une âme centrale ou "saumon" métallique 12 évidée en son centre de manière à ménager une galerie technique 14 servant notamment à faire passer et rassembler les fils de liaison aux différents capteurs (voir plus bas). Le saumon métallique 12 est enrobé d'un revêtement 16, par exemple en résine époxyde, moulé aux dimensions du gabarit normalisé. L'état de surface du revêtement 16 est réalisé lisse et sans aspérité de manière à permettre un enfilage aisé et un placement uniforme du bas de contention sur la forme 10.

Avantageusement, pour permettre par exemple l'échange d'un capteur défectueux ou la vérification de la connectique, la forme 10 est réalisée en plusieurs éléments démontables assemblés de façon étanche et sans solution de continuité, par exemple cinq éléments indépendants "haut de cuisse", "genou", "mollet", "cheville" et "pied".

L'ensemble de la forme est placé sur une bague support 18 elle-même montée sur un coffret 20 pouvant en particulier contenir l'électronique de traitement des signaux.

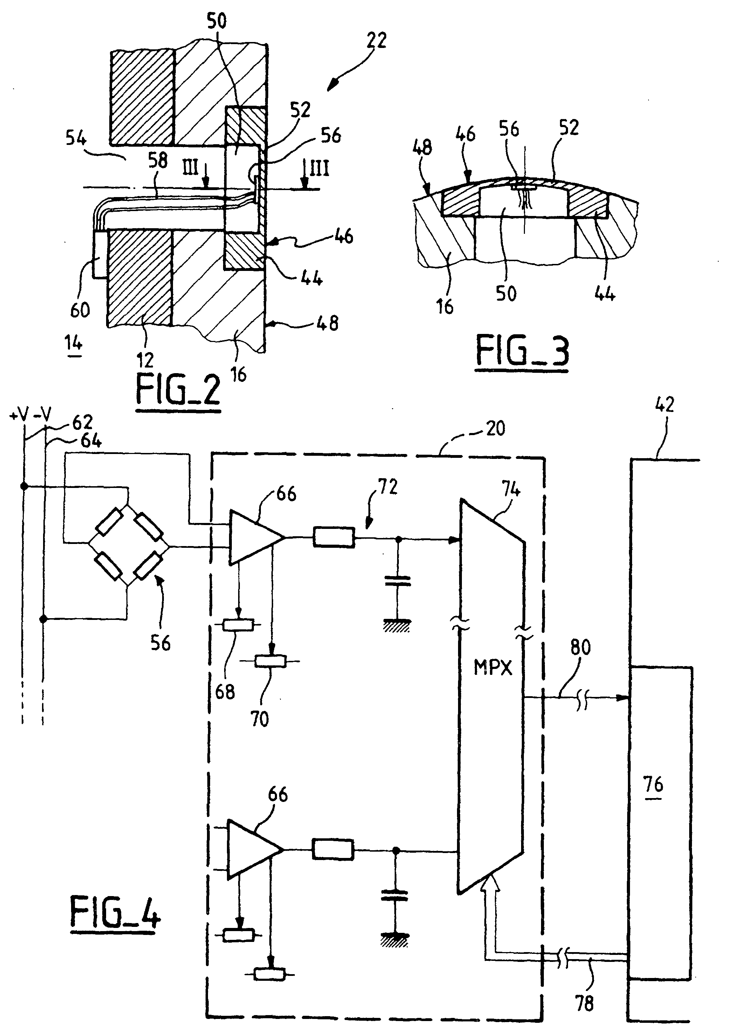

La forme 10 est pourvue d'une pluralité de capteurs 22, qui seront décrits plus en détail en référence aux figures 2 et 3, disposés à plusieurs niveaux successifs de la jambe, par exemple à sept niveaux successifs 24 à 36 , avec à chaque niveau cinq à onze capteurs répartis périphériquement. La répartition des capteurs peut être celle indiquée par le tableau ci-après, donnant un total de soixante capteurs, soit soixante mesures simultanées de pression sur sept niveaux de la jambe (la "hauteur par rapport au sol" correspond à la hauteur du capteur mesurée depuis la plante du pied dans le sens proximal).

| Niveau n° | Hauteur p.r. au sol (cm) | Nb capteurs par niveau | Écart angulaire entre capteurs (°) | Écart linéaire correspondant (cm) |
|---|---|---|---|---|
| 1 | 12 | 5 | 72 | 4,6 |
| 2 | 20 | 6 | 60 | 4,8 |
| 3 | 31 | 8 | 45 | 4,4 |
| 4 | 39 | 8 | 45 | 4,3 |
| 5 | 45 | 11 | 32,7 | 3,4 |
| 6 | 60 | 11 | 32,7 | 4,3 |
| 7 | 72 | 11 | 32,7 | 4,7 |

L'ensemble peut être monté sur un support tournant 38 du même type que celui utilisé avec les gabarits Hohenstein traditionnels pour permettre un enfilage aisé du bas.

Le coffret d'électronique 20 est relié par une liaison 40 à un micro-ordinateur 42 assurant le traitement des signaux et la visualisation des pressions mesurées.

On va maintenant décrire plus en détail la structure des capteurs, en référence aux figures 2 et 3. La figure 2 est une coupe en élévation de l'un des capteurs 22, et la figure 3 est une vue en plan, en coupe, de ce même capteur, qui permet de voir la courbure de la jambe.

Le capteur 22 est réalisé à partir d'une pastille support 44, par exemple en résine époxyde, dont la surface extérieure 46 est définie de manière à présenter localement la même courbure que le reste de la jambe et assurer une absence de solution de continuité avec la surface extérieure 48 de celle-ci. Ainsi, la forme ne présente aucune saillie ni discontinuité à l'endroit du capteur, qui ne gênera donc pas l'enfilage du bas sur la forme et, surtout, ne modifiera en aucune façon les conditions d'application de la pression sur la jambe par le bas (à la différence du dispositif de l'art antérieur où une capsule était intercalée entre bas et gabarit, modifiant donc localement les conditions d'application de la contention).

Intérieurement, la pastille 44 comporte une cavité 50, de manière à définir entre cette cavité et la surface extérieure 46 une mince paroi ou diaphragme 52, susceptible d'une microdéformation. Par "microdéformation" on entendra une modification de courbure suffisamment importante pour être mesurable, mais suffisamment faible pour ne pas modifier sensiblement, au plan macroscopique, les conditions locales d'application de la pression de contention par le bas au point de mesure.

Des dimensions typiques d'une pastille support 44 sont : diamètre extérieur : 24 mm ; diamètre intérieur de la cavité 50 : 13 mm, épaisseur de la membrane 52 : 0,75 mm ; rayon de courbure de la surface 46 : 36 à 80,5 mm selon l'emplacement du capteur. Pour mesurer la microdéformation de la paroi 56, on colle sur celle-ci, du côté de la cavité 50, une jauge extensométrique 56, par exemple un pont de quatre jauges de type jauge pour capteur de pression à diaphragme, compensée en température.

Les fils 58 d'alimentation et de mesure de la jauge 56 traversent le revêtement 16 et le saumon métallique 12 par un puits de jauge 54 débouchant dans la galerie technique 14 et permettant de relier ces fils à un connecteur primaire 60 lui même relié, ainsi que les autres connecteurs homologues, au coffret électronique 20.

La figure 4 illustre les circuits électroniques permettant de réaliser la mesure : l'une des diagonales du pont de jauges 56 est alimenté par deux tensions symétriques 62, 64 et l'autre diagonale est reliée aux deux entrées d'un amplificateur différentiel 66 dont il est possible d'ajuster individuellement le gain et l'offset par des potentiomètres respectifs 68 et 70. Le signal de sortie de l'amplificateur 66 est transmis via un filtre passe-bas 72 à un multiplexeur analogique 74 recevant en entrée les signaux délivrés par chacun des amplificateurs associés au pont de jauges de chacun des soixante capteurs considérés. Le signal de sortie du multiplexeur 74 est appliqué à l'entrée d'une carte analogique 76 du micro-ordinateur 42, qui adresse par un bus 78 le multiplexeur 74 de manière que celui-ci délivre sur une ligne 80 l'un des soixante signaux de mesure en provenance des capteurs.

La scrutation des capteurs est effectuée cycliquement et en continu, ce qui permet un affichage simultané et évolutif de l'ensemble des valeurs relevées aux différents points de mesure.

Ces valeurs peuvent être affichées sous forme numérique (valeur relative de la pression, en hPa) et également sous forme graphique, par exemple avec un graphique à barres et/ou un graphique coloré, la couleur variant par rapport au point de consigne de la pression au point de mesure considéré (par exemple vert si la pression ne s'écarte pas de plus d'un taux prédéterminé de la valeur de consigne, rouge dans le cas contraire).

Par ailleurs, les valeurs mesurées peuvent subir un certain nombre de traitements mathématiques, par exemple par application d'un modèle mathématique dont les valeurs des paramètres sont déterminées pour chaque point de mesure dans le but de compenser les effets locaux, tels que l'épaisseur des parois sensibles qui dépend des rayons de courbure de la forme à l'endroit considéré.

Pour l'ajustement des paramètres individuels de gain et d'offset de chacun des amplificateurs 66, on prévoit une étape préalable d'étalonnage des capteurs, mise en oeuvre en enfermant l'ensemble du dispositif dans une enceinte close 82 (figure 1) et en mettant cette enceinte sous une pression parfaitement calibrée : les réglages seront alors effectués de manière que tous les capteurs affichent la même valeur de pression : pression nulle en l'absence d'enceinte 82, et pression de calibrage après pressurisation de l'enceinte 82.

Ce réglage peut être effectué de plusieurs manières : réglage manuel des potentiomètres de gain et d'offset, réglage programmé des gains et des offsets depuis l'informatique du micro-ordinateur, ou encore choix d'un amplificateur qui évite de compenser.

L'invention trouve de nombreuses applications, parmi lesquelles on peut citer :
- à l'usine, le réglage des machines de tricotage et le contrôle de qualité en production,
- en clinique, comme outil d'analyse pour vérifier la conformité entre la contention réellement appliquée par l'orthèse et la contention prescrite par le praticien, ou encore pour étudier de façon dynamique les variations des pressions en fonction des mouvements du membre (en utilisant dans ce cas une forme articulée),
- dans le domaine pédagogique, pour la formation à la pose des bandes de contention : l'affichage immédiat des pressions permet de visualiser immédiatement, en cours de pose, le caractère insuffisant ou, au contraire, excessif de la tension de la bande.
- dans le domaine militaire, pour l'évaluation de l'efficacité des combinaisons dites "anti-g" qui appliquent de façon contrôlée des pressions sur certaines parties du corps des pilotes de chasse pour compenser les fortes accélérations subies au cours des manoeuvres de l'appareil.

## Revendications

1. Un dispositif pour établir une cartographie simultanée des pressions susceptibles d'être appliquées par une orthèse compressive sur un membre, notamment sur la jambe, ce dispositif comportant une forme rigide (10) apte à recevoir l'orthèse compressive, et une pluralité de capteurs de pression (22) répartis en différents points de la forme
**caractérisé en ce que** la forme reproduit le volume dudit membre,
**en ce que** la forme incorpore la pluralité de capteurs (22) et dont la surface extérieure (46) présente localement la même courbure que le reste de la forme sans solution de continuité avec la surface extérieure (48) de cette forme,
de manière que la forme ne présente aucune saillie ni discontinuité à l'endroit du capteur,
et **en ce que** chacun de ces capteurs est apte à mesurer la pression appliquée localement sur la forme par l'orthèse à l'endroit du capteur perpendiculairement à la surface de la forme.

2. Le dispositif de la revendication 1, dans lequel chacun des capteurs comprend, à l'endroit du point de mesure, un diaphragme (52) susceptible d'une microdéformation sous l'effet de la pression appliquée par l'orthèse, et des moyens de mesure de cette microdéformation.

3. Le dispositif de la revendication 2, dans lequel les moyens de mesure de la microdéformation du diaphragme comprennent un pont de jauges extensométriques (56) compensées en température.

4. Le dispositif de la revendication 2, dans lequel le diaphragme fait partie d'une pastille support rapportée (44), incorporée à la forme de manière que sa surface extérieure (46), qui comprend le diaphragme affleure celle (48) de la forme.

5. Le dispositif de la revendication 2, comprenant en outre des moyens d'étalonnage de la pluralité de capteurs, avec une enceinte close étanche (82) pressurisable enfermant la forme.

6. Le dispositif de la revendication 1, comprenant en outre des moyens (20, 42) de traitement et d'affichage des mesures recueillies par la pluralité de capteurs.

## Patentansprüche

1. Vorrichtung zur Erstellung einer simultanen Kartographie der Drücke, die durch eine kompressive Orthese auf ein Körperglied ausgeübt werden können, insbesondere ein Bein, wobei die Vorrichtung eine steife Form (10) aufweist, die geeignet ist, die Orthese zu empfangen, und eine Vielzahl von Drucksensoren (22), verteilt auf verschiedene Punkte der Form, **dadurch gekennzeichnet, dass** die Form den Rauminhalt des Körperteils reproduziert,
dadurch, dass die Vielzahl von Sensoren (22) in der Form eingebettet sind und deren äußere Oberfläche (46) lokal die selbe Krümmung wie der Rest der Form aufweist, ohne Lösung der Kontinuität mit der äußeren Oberfläche (48) dieser Form, so dass die Form weder Vorsprung noch Unterbrechung an der Stelle des Sensors aufweist,
und dadurch, dass jeder der Sensoren den Druck messen kann, der lokal auf die Form durch die Orthese an der Stelle des Sensors senkrecht zur Oberfläche der Form ausgeübt wird.

2. Vorrichtung gemäß Anspruch 1, in der jeder der Sensoren an der Stelle des Messpunkts ein Diaphragma (52) enthält, das zu einer Mikrodeformation unter dem durch die Orthese ausgeübten Druck fähig ist, und Mittel zur Messung dieser Mikrodeformation.

3. Vorrichtung gemäß Anspruch 2, in der die Mittel zur Messung der Mikrodeformation des Diaphragmas eine extensometrische, temperaturkompensierte Messbrücke (56) enthalten.

4. Vorrichtung gemäß Anspruch 2, in der das Diaphragma Teil einer aufgesetzten Trägerkapsel (44) ist, die so in der Form eingebettet ist, dass ihre äußere Oberfläche (46), die das Diaphragma enthält, mit derjenigen (48) der Form bündig abschließt.

5. Vorrichtung gemäß Anspruch 2, die darüber hinaus Mittel zur Eichung der Vielzahl von Sensoren aufweist, mit einer drückbaren, dichten, abgeschlossenen Einfassung (82), die die Form einschließt.

6. Vorrichtung gemäß Anspruch 1, die darüber hinaus Mittel (20, 42) zur Verarbeitung und Anzeige der durch die Vielzahl von Sensoren empfangenen Messungen aufweist.

## Claims

1. A device for establishing a simultaneous map of pressures that can be applied by a compressive orthosis on a limb, in particular on the leg, said device comprising a rigid former (10) suitable for receiving the compressive orthosis and a plurality of pressure sensors (22) distributed over different points of the former, **characterised in that** the former reproduces the volume of said limb,
**in that** the former (10) incorporates said plurality of sensors (22), the outer surface (46) of which locally presents the same curvature as the remainder of the former with no gap relative to the outside surface (48) of said former,
so that the former has no projections or discontinuities at the location of the sensor,
and **in that** each sensor is adapted to measure the pressure that is applied locally to the former by the orthosis at the location of the sensor and perpendicularly to the surface of the former.

2. The device of claim 1, in which each sensor comprises, at the location of the measurement point, a membrane (52) capable of being subjected to microdeformation under the effect of the pressure applied by the orthosis, and means for measuring said microdeformation.

3. The device of claim 2, in which the means for measuring the microdeformation of the membrane comprise a temperature-compensated strain gauge bridge (56).

4. The device of claim 2, in which the membrane forms a portion of a support pellet (44) fitted to the former in such a manner that its outside surface (46), which includes the membrane, is flush with the surface (48) of the former.

5. The device of claim 2, further comprising means for calibrating the plurality of sensors by means of a leakproof enclosure (82) that can be pressurized and that encloses the former.

6. The device of claim 1, further comprising means (20, 42) for processing and displaying the measurements taken by the plurality of sensors.
